# EUROPÄISCHE PATENTSCHRIFT

(11) **EP 3 600 230 B1**
(45) Veröffentlichungstag und Bekanntmachung des Hinweises auf die Patenterteilung: **08.10.2025**
(21) Anmeldenummer: 18711508.4
(22) Anmeldetag: 09.03.2018
(51) Int. Cl.: A61K 8/34, A61Q 5/02, A61Q 5/06, A61K 8/73, A61K 8/02, A61K 8/04

(54) **SPRÜHBARES KOSMETISCHES MITTEL II**
SPRAYABLE COSMETIC AGENT II
AGENT COSMÉTIQUE PULVÉRISABLE II

(30) Priorität: 31.03.2017 DE 102017205556; 21.11.2017 DE 102017220775
(43) Veröffentlichungstag der Anmeldung: 05.02.2020
(73) Patentinhaber: Henkel AG & Co. KGaA, 40589 Düsseldorf (DE)
(72) Erfinder: METTEN, Diane, 22393 Hamburg (DE); SCHEFFLER, Rene, 25479 Ellerau (DE); LANGE, Julia Bibiane, 24576 Bad Bramstedt (DE); MARTINEZ, Cyrielle, 22763 Hamburg (DE); KNAPPE, Thorsten, 22869 Schenefeld (DE); BAYERSDÖRFER, Rolf, 22589 Hamburg (DE)
(86) Internationale Anmeldenummer: PCT/EP2018/055892
(87) Internationale Veröffentlichungsnummer: WO 2018/177721

(56) Entgegenhaltungen:
- WO-A1-2017/178855
- DE-A1- 19 640 099
- JP-A- H11 158 034
- US-A1- 2012 282 190
- ANONYMOUS: "D.S.A 7 RED", 4 October 2016 (2016-10-04), XP055469654, Retrieved from the Internet <URL:http://www.agrana.com/fileadmin/inhalte/agrana_group/2017/images/Starch/Folder/Kosmetik/PDB_D.S.A.7_9017_en.pdf> [retrieved on 20180423]
- ANONYMOUS: "Dry Shampoo black & conditioner with D.S.A.7 black 20%", 5 December 2016 (2016-12-05), XP055469665, Retrieved from the Internet <URL:https://services.agrana.com/fileadmin/inhalte/austria/products/Kosmetik_Formulierungen/2018_Kosmetikdatein_Download/2018_Formulierungen/AGRANA_-_Dry_Shampoo_black_and_conditioner_with_D.S.A._7_black_20.pdf> [retrieved on 20180423]

## Beschreibung

Die vorliegende Anmeldung betrifft sprühbare kosmetische Zusammensetzung zur Behandlung keratinischer Fasern, welche neben einem Lösungsmittel und einem Treibmittel weiterhin Fasern enthalten. Diese Mittel eignen sich für vielfältige kosmetische Zwecke, einschließlich der Reinigung, Auffrischung und Desodorierung keratinischer Fasern.

Weiterhin betrifft die Anmeldung die kosmetische Verwendung dieser Mittel und haarkosmetische Verfahren unter Einsatz dieser Mittel.

Eine ansprechend aussehende Frisur wird heute allgemein als unverzichtbarer Teil eines gepflegten Äußeren angesehen. Ein wesentlicher Aspekt der Haarpflege ist die Auffrischung und Reinigung der Haare.

Im Bereich der Reinigung, Auffrischung und Desodorierung keratinhaltiger Fasern hat die Sprühapplikation fester kosmetischer Wirkstoffe eine gewisse Bedeutung. Bei diesen Wirkstoffen handelt es sich in der Regel um Stärke oder Stärkederivate, welche aufgrund ihres Fettaufnahmevermögens als wesentlicher Bestandteil von Trockenshampoos auf das Haar aufgebracht und anschließend auf dem Haar belassen oder ausgebürstet werden.

Wie bei anderen Sprühapplikationen auch, besteht bei der Sprühapplikation feststoffhaltiger Trockenshampoos die technische Schwierigkeit, einen gleichmäßigen Auftrag des Sprühkosmetikums zu gewährleisten. Zur Lösung dieses Problems wurden in der Vergangenheit insbesondere spezifische Ausgestaltungen der Sprühvorrichtung einschließlich des Sprühkopfs beschrieben.

Aus der JP H11 158034 A sind Aerosol-Zubereitungen mit Mikrofasern bekannt, die auf die Haare gesprüht werden, um ihnen mehr "Dicke" zu verleihen.

Die US 2012/282190 A offenbart Trockenshampoozusammensetzungen, umfassend einen Alkohol und ein Treibmittel.

Die Aufgabe der vorliegenden Erfindung war es nun, sprühbare Haarkosmetika bereitzustellen, welche sich gegenüber herkömmlichen Zubereitungen durch verbesserte Applikationseigenschaften, insbesondere ein verbessertes Sprühbild auszeichnen. Es hat sich gezeigt, dass diese Aufgabe durch den Zusatz von Fasern zu den sprühbaren kosmetischen Zubereitungen gelöst werden kann.

Durch die vorliegende Erfindung wird bereitgestellt:
Geänderte Beschreibung
   1. Eine kosmetische Zusammensetzung zur kosmetischen Behandlung keratinischer Fasern, umfassend- jeweils bezogen auf ihr Gesamtgewicht -
      (a) 0,1 bis 2,0 Gew.-% Fasern
      (b) 5,0 bis 19 Gew.-% Ethanol
      (c) 78 bis 94 Gew.-% Treibmittel,
      wobei die Zusammensetzung 0,1 bis 2,0 Gew.-% hydrophobierte oder hydrophilierte Fasern enthält.
   2. Kosmetische Zusammensetzung nach Punkt 1, wobei sie ferner
      (d) 0 bis 5 Gew.-% Farbstoff
      (e) (e) 0 bis 5 Gew.-% Pigment(e) enthält, mit der Maßgabe, dass die Summe der Mengen der Inhaltsstoffe
      (d) und (e) 0,0001 bis 10 Gew.-%, bezogen auf das Gesamtgewicht der kosmetischen Zusammensetzung, beträgt.
   3. Kosmetische Zusammensetzung nach Punkt 1 oder 2, wobei der Gewichtsanteil der Fasern am Gesamtgewicht der kosmetischen Zusammensetzung 0,1 bis 1 ,0 Gew.-% und insbesondere 0,1 bis 0,5 Gew.-% beträgt.
   4. Kosmetische Zusammensetzung nach einem der vorherigen Punkte, wobei die Fasern ausgewählt sind aus der Gruppe der aus natürlichen Fasermaterialien hergestellten Fasern.
   5. Kosmetische Zusammensetzung nach einem der vorherigen Punkte, wobei die Fasern ausgewählt sind aus der Gruppe der pflanzlichen Fasern, insbesondere der Cellulosefasern, vorzugsweise der Baumwollfasern, Flachsfasern und Kapopkfasern.
   6. Kosmetische Zusammensetzung nach einem der vorherigen Punkte, wobei der Gewichtsanteil des Ethanols am Gesamtgewicht der kosmetischen Zusammensetzung 7,0 bis 17 Gew.-%, vorzugsweise 9,0 bis 15 Gew.-% und insbesondere 10 bis 14 Gew.-% beträgt.
   7. Kosmetische Zusammensetzung nach einem der vorherigen Punkte, wobei die Zusammensetzung als weiteren Bestandteil mindestens eine Stärkeverbindung umfasst.
   8. Kosmetische Zusammensetzung nach einem der vorherigen Punkte, wobei die Zusammensetzung bezogen auf ihr Gesamtgewicht 1 ,0 bis 10 Gew.-%, vorzugsweise 2,0 bis 6,0 Gew.-% und insbesondere 3,0 bis 5,0 Gew.-% einer Stärkeverbindung umfasst.
   9. Kosmetische Zusammensetzung nach einem der vorherige Punkte 2 bis 7, wobei sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf ihr Gesamtgewicht, Farbstoff(e) enthält.
   10. Kosmetische Zusammensetzung nach einem der vorherigen Punkt 2 bis 8, wobei sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf ihr Gesamtgewicht, Pigment(e) enthält.
   11. Kosmetische Zusammensetzung nach einem der vorherigen Punkt 2 bis 9, wobei sie mindestens ein Pigment aus der Gruppe CI 12490, CI 14700, CI 14720, C115510, C115985, CI45380, CI47005, CI60730, C161565, CI73360, CI74160, CI77007, CI77019, CI77288, CI77289, CI77491 , CI77492 enthält, wobei die Gesamtmenge an Pigment(en) aus dieser Gruppe 0,0001 bis 10 Gew.- %, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf das Gesamtgewicht der kosmetischen Zusammensetzung, beträgt.
   12. Kosmetische Zusammensetzung nach Punkt 2 und einem der vorherigen Punkte 6 bis 10, wobei sie den Inhaltsstoff (d) und/oder (e) in Form eines Stärke-Farbstoff-Compounds und/oder Stärke-Pigment-Compounds, vorzugsweise in Form von gefärbter und/oder pigmentierter Reisstärke, enthält.
   13. Kosmetische Zusammensetzung nach einem der vorherigen Punkte 2 bis 11, wobei sie die Inhaltsstoffe (a) und (d) und/oder (a) und (e) in Form eines Faser-Farbstoff-Compounds oder Faser-Pigment-Compounds, vorzugsweise in Form von gefärbten und/oder pigmentierten Fasern, enthält.
   14. Kosmetisches Mittel, umfassend a) eine kosmetische Zusammensetzung nach einem der Punkte 1 bis 12 b) einen Aerosolabgabebehälter.
   15. Verwendung einer kosmetischen Zusammensetzung nach einem
      der Punkte 1 bis 12 zur Reinigung keratinischer Fasern.

Die kosmetische Zusammensetzung enthält als ihren ersten wesentlichen Bestandteil Fasern. Der Zusatz dieser Fasern beeinflusst in nicht zu erwartender Weise die Applikationseigenschaften des sprühbaren Haarkosmetikums. Das Sprühbild und der Auftrag des versprühten Kosmetikums werden gleichmäßiger. In einer standardisierten Versuchsanordnung bewirkt der Zusatz der Fasern bei gleichem Druck in der Sprühvorrichtung und gleichem Abstand zu der zu besprühenden Fläche zudem eine überraschende Vergrößerung der erzielten Sprühfläche.

Als Fasern werden gestreckte, flexible Gebilde aus Fasermaterial mit einem Verhältnis von Länge zu Durchmesser oberhalb 3:1, vorzugsweise oberhalb 5:1 und insbesondere oberhalb 10:1. Fasern können in Längsrichtung keine Druck-, sondern nur Zugkräfte aufnehmen.

Die Länge bevorzugt eingesetzter Fasern liegt im Bereich von 1,0 bis 200 µm, vorzugsweise von 2,0 bis 150 µm und insbesondere von 5,0 bis 100 µm. Selbstverständlich sind aber auch Fasern größerer Faserlänge, beispielsweise Fasern mit einer Länge oberhalb 200µm oder oberhalb 300µm einsetzbar.

Das Fasermaterial kann natürlichen Ursprungs sein. Als natürliche Fasern werden all jene Fasern bezeichnet, welche ohne eine chemische Veränderung aus pflanzlichem, tierischem oder mineralischem Material gewonnen werden.

Pflanzenfasern kommen bei Pflanzen als Leitbündel im Stängel, im Stamm, in der Rinde oder als Samen-Fortsätze vor. Pflanzenfasern bestehen in der Regel zum überwiegenden Teil aus Cellulose. Entsprechende Fasern werden unter der Bezeichnung Cellulosefasern zusammengefasst.

Einer verbreiteten Unterteilung gemäß wird bei den Pflanzenfasern zwischen Samenfasern, Bastfasern, Blattfasern und Fruchtfasern unterschieden. Zur Gruppe der Samenfasern zählen beispielsweise die Baumwollfasern, Kapok, Akon oder Pappelflaum. Die Gruppe der Bastfasern schließt u.a. Bambusfaser, Fasernessel, Hanffaser, Jute und Flachsfaser mit ein. Sisal ist dem gegenüber den Blattfasern zuzurechnen, während die Kokosfasern zur Gruppe der Fruchtfasern gehören.

Bei den tierischen Fasern kann u.a. zwischen Fasern aus Spinndrüsen und aus Haarfolikeln abstammenden Fasern unterschieden werden. Zu den Fasern aus Spinndrüsen zählt beispielsweise die Seide. Die Gruppe der aus Haarfolikeln abstammenden Fasern umfasst u.a. Wolle, Alpaka, Kamelhaar, Angora, Kaschmir, Mohair, Yakhaar, Ziegenhaar, Rinderhaar oder Rosshaar.

Besonders bevorzugt ist der Einsatz von Cellulosefasern, insbesondere von Baumwollfasern, Flachsfasern und Kapopkfasern. Bevorzugt ist weiterhin der Einsatz von tierischen Fasern aus der Gruppe der Seidenfasern, Kaschmirfasern und Wollfasern. Besonders bevorzugt ist der Einsatz von Cellulosefasern.

Als für die technische und kosmetische Wirkung besonders vorteilhaft haben sich Gewichtsanteile der Fasern am Gesamtgewicht der kosmetischen Zusammensetzung von 0,1 bis 2,0 Gew.-%, vorzugsweise von 0,1 bis 1,0 Gew.-% und insbesondere von 0,1 bis 0,5 Gew.-% erwiesen.

Als ihren zweiten wesentlichen Bestandteil enthalten die kosmetischen Zusammensetzungen Ethanol. Der Gewichtsanteil des Ethanols am Gesamtgewicht der kosmetischen Zusammensetzung beträgt vorzugsweise 7,0 bis 17 Gew.-%, bevorzugt 9,0 bis 15 Gew.-% und insbesondere 10 bis 14 Gew.-%. Die Fasern sind im Ethanol suspendiert.

Ein dritter wesentlicher Bestandteil der kosmetischen Zusammensetzungen ist das Treibmittel. Als kosmetische und technisch besonders vorteilhaft hat es sich erwiesen, wenn der Gewichtsanteil des Treibmittels am Gesamtgewicht der Zusammensetzung 82 bis 92 Gew.-%, vorzugweise 84 bis 90 Gew.-% beträgt.

Bevorzugte Treibmittel (Treibgase) sind ausgewählt aus Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Bevorzugt ist der Einsatz von Propan, Propan/Butan-Gemischen oder Dimethylether, besonders bevorzugt der Einsatz von Propan/Butan-Gemischen.

Die Zusammensetzung einiger bevorzugter kosmetischer Zusammensetzungen kann der folgenden Übersicht 1 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 82 bis 92 | 80 bis 90 | 80 bis 90 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Propan/Butan | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Propan/Butan | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Propan/Butan | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Propan/Butan | 80 bis 94 | 80 bis 94 | 80 bis 92 | 80 bis 90 | 80 bis 90 |
| Optionale Additive | ad 100 | ad 100 | ad 100 | ad 100 | ad 100 |

Besonders bevorzugte kosmetische Zusammensetzungen enthalten mindestens eine farbgebende Verbindung aus der Gruppe der Farbstoffe und/oder Pigmente. Dabei enthalten die kosmetischen Zusammensetzungen
(d) 0 bis 10 Gew.-% Farbstoff(e) und/oder
(e) 0 bis 10 Gew.-% Pigment(e),
mit der Maßgabe, dass die Summe der Mengen der Inhaltsstoffe (d) und (e) 0,0001 bis 10 Gew.-% beträgt.

In anderen Worten können die kosmetischen Zusammensetzungen 0 Gew.-% Farbstoffe enthalten (dann muß mindestens ein Pigment in einer Menge von mindestens 0,0001 Gew.-% vorhanden sein), der Farbstoffgehalt kann aber auch 10 Gew.-% betragen (dann kann kein Pigment mehr vorhanden sein. Unabhängig davon, ob
- nur ein Farbstoff,
- mehrere Farbstoffe,
- nur ein Pigment,
- mehrere Pigmente,
- ein Farbstoff und mehrere Pigmente
- mehrere Farbstoffe und ein Pigment
- mehrere Farbstoffe und mehrere Pigmente
eingesetzt werden, beträgt die Gesamtmenge aller Farbstoffe und Pigmente (auch als Gesamtmenge farbgebender Komponenten bezeichnet) in der kosmetischen Zusammensetzung, bezogen auf das Gesamtgewicht der kosmetischen Zusammensetzung, 0,0001 bis 10 Gew.-%.

Bevorzugte kosmetische Zusammensetzungen sind dadurch gekennzeichnet, dass sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf das Gesamtgewicht der kosmetischen Zusammensetzung, Farbstoff(e) enthalten.

Farbstoffe sind Farbmittel, also farbgebende Substanzen, die im Gegensatz zu Pigmenten im Anwendungsmedium löslich sind (wie in Wasser, Ölen oder anderen Lösungsmitteln).

Je nach gewünschter Farbe, Farbintensität und Echtheitseigenschaften der resultierenden Färbung kann ein Farbstoff oder eine Mischung von Farbstoffen ausgewählt werden. Dabei sind alle auf dem Gebiet der Kosmetik üblichen und kommerziell erhältlichen Farbstoffe im Rahmen der vorliegenden Erfindung problemlos einsetzbar.

Ebenfalls bevorzugte kosmetische Zusammensetzungen sind dadurch gekennzeichnet, dass 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf das Gesamtgewicht der kosmetischen Zusammensetzung, Pigment(e) enthalten.

Grundsätzlich eigenen sich alle Arten von wasserunlöslichen Pigmenten, beispielsweise natürliche anorganische Pigmente (auch als Mineralpigmente bezeichnet). Diese Pigmente beinhalten überwiegend Sulfide und Oxide. Beispiele für derartige Pigmente sind Ocker (Fe(OOH); Pigment Yellow 43), gebrannte Siena (Fe₂O₃; Pigment Red 102), Umbra (Fe₂O₃ x MnO₂; Pigment Brown 7:x), Zinnober (β-HgS, PR 106), Lapislazuli (Ultramarin, Na₆Al₆Si₆O₂₄ x Na₂Sn; Pigment Blue 29), Azurit (basisches Kupfercarbonat, Cu₃[OH/CO₃]₂; PB 30), Grünerde (FeO-haltiges Silicat; Pigment Green 23), Malachit (Cu₂[(OH)₂, CO₃]) und Kohlenschwarz (Kohlenstoff (Graphit), Pigment Black 9). In Bezug auf die Vermeidung von unerwünschten sichtbaren Rückständen oder Grauschleiern bzw. die wasserbeständige temporäre Färbung der Fasern hat sich jedoch der Einsatz von synthetischen anorganischen Pigmenten als vorteilhaft erwiesen. Synthetische anorganische Pigmente werden beispielsweise durch chemische und/oder physikalische Umwandlung (Aufschluss, Fällung, Glühen) hergestellt. Hierzu zählen insbesondere
- Weißpigmente (Titandioxid (TiO₂), Pigment White PW 6; Zinksulfid (ZnS), PW 7; Zinkoxid (ZnO), PW 4; Antimonweiß (Sb₂O₃), PW 11; Lithopone (ZnS/BaSO₄), PW 5; Bleiweiß (2PbCO₃ x Pb(OH)₂), PW 1),
- untergeordnet Weiß-Füllstoffe (Calciumcarbonat, PW 18; Talkum, PW 26 und Bariumsulfat, PW 21);
- Schwarzpigmente (Manganschwarz, Spinellschwarz sowie Pigmentruße (Graphit-Kohlenstoff);
- Glanzpigmente (Absorptionspigmente, Metallpigmente oder Metalleffektpigmente und Perlglanzpigmente) sowie
- anorganische Buntpigmente (Eisenoxid-Pigmente, Eisenblau-Pigmente, Ultramarin-Pigmente sowie die aufgrund ihrer toxikologischen Eigenschaften weniger geeigneten Bleichromat-Pigmente, Chromoxid-Pigmente, Cadmium-Pigmente und Bismutvanadat-Pigmente).

Bevorzugte synthetische anorganische Pigmente sind Metallpigmente oder Metalleffektpigmente aus pulverigen Metallen oder Metalllegierungen, wie Aluminiumbronzen (Metall: Al), Goldbronzen (Metall: Cu, Cu-Al- oder Cu-Zn-Legierung), Silberbronzen (Metall: Cu-Zn-Ni), feuergefärbte Bronzen (Metall: oxidiertes Cu-Zn) sowie Patentbronzen (Metall: Cu-Zn-(Ni) + Farbstoff).

Weitere bevorzugte synthetische anorganische Pigmente sind Perlglanzpigmente, welche aus mehreren Schichten mit unterschiedlicher Brechzahl bestehen. Beispiele für derartige Perlglanzpigmente sind Magnesiumstearat, Zinkstearat und Lithiumstearat oder Ethylenglycoldistearat bzw. Polyethylenterephthalat sowie Perlglanzpigmente, welche im Wesentlichen aus Glimmer, Titandioxid (Titandioxid-Glimmer), Bismutchloridoxid oder Guanin bestehen, und darüber hinaus mit farbigen Oxidschichten (z. B. Eisenoxiden oder Chromoxide) überzogen sein können. Perlglanzpigmente auf Glimmer-Basis und auf Glimmer/Metalloxid-Basis sind dabei besonders bevorzugte Perlglanzpigmente. Glimmer gehören zu den Schicht-Silikaten. Die wichtigsten Vertreter dieser Silikate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäß bevorzugte Perlglanzpigmente erhalten. Diese Perlglanzpigmentarten weisen neben einem glitzernden optischen Effekt zusätzlich Farbeffekte auf. Des Weiteren können die erfindungsgemäß verwendbaren Perlglanzpigmente zusätzlich ein Farbpigment enthalten, welches sich nicht von einem Metalloxid ableitet.

Ganz besonders bevorzugte Perlglanzpigmente sind Pigmente, welche von der Firma Merck unter den Handelsnamen Colorona^{®} vermarktet werden, wobei die Pigmente Colorona^{®} red-brown (47-57 Gew.% Muscovit Mica (KH₂(AlSiO₄)₃), 43-50 Gew.% Fe₂O₃ (INCI: Iron Oxides CI 77491), <3 Gew.% TiO₂ (INCI: Titanium Dioxide CI 77891), Colorona^{®} Blackstar Blue (39-47 Gew.% Muscovit Mica (KH₂(AlSiO₄)₃), 53-61 Gew.% Fe₃O₄ (INCI: Iron Oxides CI 77499)), Colorona^{®} Siena Fine (35-45 Gew.% Muscovit Mica (KH₂(AlSiO₄)₃), 55-65 Gew.% Fe₂O₃ (INCI: Iron Oxides CI 77491)), Colorona^{®} Aborigine Amber (50-62 Gew.% Muscovit Mica (KH₂(AlSiO₄)₃), 36-44 Gew.% Fe3O4 (INCI: Iron Oxides CI 77499), 2-6 Gew.% TiO₂ (INCI: Titanium Dioxide CI 77891)), Colorona^{®} Patagonian Purple (42-54 Gew.% Muscovit Mica (KH₂(AlSiO₄)₃), 26-32 Gew.% Fe₂O₃ (INCI: Iron Oxides CI 77491), 18-22 Gew.% TiO₂ (INCI: Titanium Dioxide CI 77891), 2-4 Gew.% Preussisch Blau (INCI: Ferric Ferrocyanide CI 77510)), Colorona^{®} Chameleon (40-50 Gew.% Muscovit Mica (KH₂(AlSiO₄)₃), 50-60 Gew.% Fe₂O₃ (INCI: Iron Oxides CI 77491)) und Silk^{®} Mica ( >98 Gew.% Muscovit Mica (KH₂(AlSiO₄)₃)) besonders bevorzugt sind.

Eine Gruppe besonders bevorzugter Pigmente bilden die farbgebenden synthetischen Eisenoxide. Besonders bevorzugte Vertreter dieser Substanzklasse sind Pigment Brown 6 (CI No 77491), Pigment Red 101 (CI No 77491), Pigment Yellow 42 (CI No 77492), Pigment Black 11 (CI No 77499) sowie Mischungen dieser Pigmente.

Ganz besonders bevorzugt einsetzbare Pigmente zeichnen sich durch eine gute Versprühbarkeit in den erfinungsgemäßen Zusammensetzungen, ein homogenes Sprühbild sowie eine äußerst geringe Neigung zum Verstopfen der Sprühdüsen ("clogging") aus. Hier sind äußerst bevorzugte kosmetische Zusammensetzungen dadurch gekennzeichnet, dass sie mindestens ein Pigment aus der Gruppe CI12490, CI14700, CI14720, C115510, CI15985, CI45380, CI47005, CI60730, CI61565, CI73360, CI74160, CI77007, CI77019, CI77288, CI77289, CI77491, CI77492 enthalten, wobei die Gesamtmenge an Pigment(en) aus dieser Gruppe 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf das Gesamtgewicht der kosmetischen Zusammensetzung, beträgt.

Bevorzugte kosmetische Zusammensetzungen enthalten als weiteren Bestandteil mindestens eine Stärkeverbindung. Unter der Bezeichnung "Stärke" wird dabei ist ein Reservekohlenhydrat verstanden, welches von vielen Pflanzen in Form von üblicherweise 1 bis 200 µm großen Stärkekörnern (Granula) in verschiedenen Pflanzenteilen, z.B. in Knollen oder Wurzeln, Getreidesamen, Früchten sowie im Mark gespeichert wird.

Vorzugsweise liegt die Stärkeverbindung als in dem Ethanol dispergierte Feststoffpartikel vor. Unter "Feststoffpartikel" werden bei 20°C und 1013,25 mbar partikulär vorliegende Feststoffe verstanden.

Eine erfindungsgemäß bevorzugt verwendbare Stärkeverbindung wird ausgewählt unter mindestens einem - gegebenenfalls modifizierten - Polykondensationsprodukt von D-Glucose erhalten aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok. Besonders bevorzugt enthält die erfindungsgemäße Zusammensetzung mindestens eine Stärkeverbindung, die Tapioka Stärke, Kartoffelstärke, Maisstärke oder Reisstärke ist oder sich davon ableitet. Gemische der vorgenannten Stärkeverbindungen sind erfindungsgemäß ebenso umfasst.

Ganz besonders bevorzugt ist der Einsatz von Reisstärke. Stärkeverbindungen auf Grundlage von Reisstärke sind beispielsweise unter den Bezeichnung Remy DR KA (INCI Bezeichnung: Oryza Sativa (Rice) Starch, CAS Nummer 9005-25-8) von der Firma Bene O Remy Industries oder unter der Bezeichnung Rice Starch D.S.A. 7 (INCI Bezeichnung: Oryza Sativa (Rice) Starch, Cetrimonium Chloride; CAS Nummer 9005-25-8) von der Firma Agrana erhältlich.

In Bezug auf die Reinigungsleistung hat sich der Einsatz von nativer und/oder physikalisch modifizierter Reisstärke als besonders vorteilhaft erwiesen. Unter nativer Stärke wird eine Stärke verstanden, welche aus Stärke-haltigen Pflanzen isoliert wird und welche nach der Isolation und Aufreinigung weder physikalisch noch chemisch modifiziert wurde. Hingegen wird unter physikalisch modifizierter Stärke eine Stärke verstanden, welche nach der Isolierung mindestens einer physikalischen Modifikation unterworfen wurde. Unter physikalischer Modifikation ist hierbei die Modifikation unter Anwendung von Druck und/oder Hitze und/oder Licht zu verstehen. Eine Modifikation mittels chemischer und enzymatischer Reaktionen, beispielsweise die Hydrolyse der Stärke, fällt hierbei jedoch nicht unter den Begriff der physikalischen Modifikation. Eine bevorzugt eingesetzte physikalische Modifikation ist die Anwendung von Hitze, insbesondere das Kochen der nativen Stärke. Bevorzugte Ausführungsformen der vorliegenden Erfindung sind daher dadurch gekennzeichnet, dass die Zusammensetzungen mindestens eine Stärke, ausgewählt aus chemisch und/oder physikalisch modifizierten Reisstärken, insbesondere aus physikalisch modifizierter Reisstärke, enthalten. Durch den Einsatz von nativen und/oder physikalisch modifizierten Reisstärken in Kombination mit Farbstoffen und/oder Pigmenten wird eine besonders hohe Haftung der Farbstoffe und/oder Pigmente auf der Stärke erzielt, so dass neben der hervorragenden Reinigungsleistung auch eine lang anhaltende temporäre Haarfärbung ermöglicht wird.

Hierdurch ist es möglich und bevorzugt, vorgefärbte und/oder vorpigmentierte Stärken einzusetzen, die sich mit den kosmetischen Zusammensetzungen herausragend versprühen lassen. Besonders bevorzugte kosmetische Zusammensetzung sind daher dadurch gekennzeichnet, dass sie den Inhaltsstoff (d) und/oder (e) in Form eines Stärke-Farbstoff-Compounds und/oder Stärke-Pigment-Compounds, vorzugsweise in Form von gefärbter und/oder pigmentierter Reisstärke, enthalten.

Es hat sich zusätzlich herausgestellt, dass sich die Zusammensetzung der Stärkepartikel selbst als für die kosmetische Wirkung relevant erwiesen hat. Daher weisen bevorzugt eingesetzte Partikel einen bestimmten Anteil der nativen und/oder physikalisch modifizierten Stärke, insbesondere Reisstärke, auf. Es ist somit bevorzugt, wenn das Stärke-Farbstoff-Compound und/oder Stärke-Pigment-Compound mindestens eine Stärke, insbesondere eine physikalisch modifizierte Reisstärke, in einer Gesamtmenge von 70 bis 96 Gew.-%, insbesondere von 80 bis 94 Gew.-%, jeweils bezogen auf das Gesamtgewicht des Partikels (Compounds), enthält. Der Einsatz von Partikeln, welche einen hohen Gewichtsanteil an physikalisch modifizierter Reisstärke enthalten, führt zu besonders homogenen Sprühbildern und bevorzugte Haftung der Farbe auf den keratinischen Fasern.

Zusätzlich zu solchen Compounds aus Stärke und farbgebenden Substanzen oder an ihrer Stelle können naturgemäß auch die Fasern der kosmetischen Zusammensetzungen vorgefärbt und/oder vorpigmentiert werden. Hier sind kosmetische Zusammensetzungen besonders bevorzugt, die die Inhaltsstoffe (a) und (d) und/oder (a) und (e) in Form eines Faser-Farbstoff-Compounds oder Faser-Pigment-Compounds, vorzugsweise in Form von gefärbten und/oder pigmentierten Fasern, enthalten.

Die kosmetische Wirkung und Applizierbarkeit der kosmetischen kann durch den Stärkegehalt vorteilhaft beeinflusst werden. Bevorzugte kosmetische Zusammensetzungen sind daher dadurch gekennzeichnet, dass diese Zusammensetzung bezogen auf ihr Gesamtgewicht 1,0 bis 10 Gew.-%, vorzugsweise 2,0 bis 6,0 Gew.-% und insbesondere 3,0 bis 5,0 Gew.-% einer Stärkeverbindung umfassen.

Die Zusammensetzung einiger weiterer bevorzugter kosmetischer Zusammensetzungen kann der folgenden Übersicht 2 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Propan/Butan | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Propan/Butan | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Propan/Butan | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Propan/Butan | 78 bis 92 | 78 bis 90 | 78 bis 88 | 78 bis 88 | 78 bis 88 |
| Optionale Additive | ad 100 | ad 100 | ad 100 | ad 100 | ad 100 |

Die eingangs als erster wesentlicher Bestandteil der kosmetischen Zusammensetzungen beschriebenen Fasern können in unterschiedlicher Weise modifiziert werden. Derartige Modifikationen betreffen beispielsweise die weiter oben beschriebene Länge der Fasern oder deren Längen zu Durchmesser Verhältnis.

Eine weitere Möglichkeit, die technischen und kosmetischen Eigenschaften der Zusammensetzungen zu beeinflussen liegt in der Modifikation der Oberflächeneigenschaften der eingesetzten Fasern.

In einer ersten bevorzugten Ausführungsform werden hydrophobierte Fasern natürlichen Ursprungs eingesetzt. Die Hydrophobierung (Imprägnierung) bewirkt eine wasserabstoßende Ausrüstung der Faser. Als Hydrophobierungsmittel eignen sich beispielsweise Paraffine oder Wachse aber auch filmbildende Silikone.

In Abhängigkeit vom Verwendungszweck der kosmetischen Zusammensetzung kann es von Vorteil sein, wenn die Fasern eine gewisse Ölabsorptionskapazität aufweisen. Diese Ölabsorptionskapazität kann mittels der zuvor beschriebenen Hydrophobierung der Fasern beeinflusst werden. Bevorzugt ist der Einsatz hydrophobierter oder nicht hydrophobierter Fasern mit einer Ölabsorptionskapazität oberhalb 0,2 g Jojobaöl pro Gramm Fasern. Besonders bevorzugt ist der Einsatz von Fasern mit einer Ölabsorptionskapazität (20°C) von 0,2 bis 0,8 g Jojobaöl/g, vorzugsweise von 0,3 bis 0,7 g Jojobaöl/g, besonders bevorzugt von 0,4 bis 0,6 g Jojobaöl/g.

Die Hydrophobierung der Fasern verbessert u.a. deren Sebum-Aufnahmevermögen. Entsprechende Fasern werden daher insbesondere in reinigenden kosmetischen Zusammensetzungen eingesetzt.

Beispielsweise um eine gleichmäßige Suspension der Fasern in der kosmetischen Zusammensetzung zu ermöglichen oder die kosmetische Wirkung der Zusammensetzung zu steigern, können die Zusammensetzungen als weiteren optionalen Bestandteil Tensid umfassen. Bevorzugt ist der Einsatz nichtionischer oder kationischer Tenside.

Bevorzugte kationische Tensind sind ausgewählt aus der Gruppe der quartären Ammoniumverbindungen. Bevorzugte quartäre Ammoniumverbindungen sind wiederum Ammoniumhalogenide, insbesondere Chloride und Bromide, wie Alkyltrimethylammoniumchloride, Dialkyldimethylammoniumchloride und Trialkylmethylammoniumchloride, z. B.

Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Ganz besonders bevorzugt ist der Einsatz von Cetyltrimethylammoniumchlorid.

Der Gewichtsanteil des Tensids am Gesamtgewicht der kosmetischen Zusammensetzung beträgt vorzugsweise 0,05 bis 2,0 Gew.-%, bevorzugt 0,1 bis 1,5 Gew.-% und insbesondere 0,2 bis 1,0 Gew.-%.

Die Zusammensetzung einiger weiterer bevorzugter kosmetischer Zusammensetzungen kann der folgenden Übersicht 3 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophobierte Faser natürlichen Ursprungs | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophobierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophobierte Cellulosefaser mit einer | 0,1 bis 5,0 | 0,1 bis 2,0 | 0,1 bis 2,0 | 0,1 bis 1,0 | 0,1 bis 0,5 |
| Faserlänge zwischen 1,0 und 200 µm | | | | | |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophobierte Faser natürlichen Ursprungs | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophobierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

In einer alternativen Ausführungsform werden in den kosmetischen Mitteln hydrophilierte Fasern eingesetzt. Entsprechende Fasern verfügen gegenüber den nicht hydrophilierten Fasern über ein erhöhtes Wasseraufnahmevermögen.

Die Wasseradsorptionskapazität (20°C) bevorzugter Fasern, insbesondere bevorzugter hydrophilierter Fasern, liegt vorzugsweise oberhalb 5,0 Gew.-%, vorzugsweise oberhalb 7,0 Gew.-% und insbesondere oberhalb 9,0 Gew.-% des Eigengewichts der Fasern.

Die Hydrophophilierung der Fasern verbessert u.a. deren Wasser-Aufnahmevermögen. Entsprechende Fasern werden daher insbesondere in auffrischenden kosmetischen Zusammensetzungen eingesetzt.

Die Zusammensetzung einiger weiterer bevorzugter kosmetischer Zusammensetzungen kann der folgenden Übersicht 4 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophilierte Cellulosefaser mit einer | 0,1 bis 5,0 | 0,1 bis 2,0 | 0,1 bis 2,0 | 0,1 bis 1,0 | 0,1 bis 0,5 |
| Faserlänge zwischen 1,0 und 200 µm | | | | | |
| Stärkeverbindung | 1,0 bis 10 | 1,0 bis 10 | 2,0 bis 6,0 | 2,0 bis 6,0 | 3,0 bis 5,0 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

Zur Verstärkung des auffrischenden Effekts kosmetischer Zusammensetzungen, kann diesen als weiterem optionalen Bestandteil mindestens ein Kühlwirkstoff zugesetzt werden.

Zur Gruppe der kosmetisch akzeptablen Komponenten, die einen Kühleffekt aufweisen, zählen Substanzen, welche durch Verdampfung (z.B. Isopropanol), oder durch Einwirkung auf Rezeptoren der Haut eine kühlende Wirkung haben. Beispiele für Substanzen aus der letztgenannten Gruppe sind die Verbindungen mit den INCI Bezeichnungen Menthol, Menthyl Lactat, Menthone Glycerin Acetal und Methoxyphenyl Menthane Carboxamide. Menthyl Ethylamido Oxalate ist im Handel beispielsweise unter der Bezeichnung Frescolat^{®} X-Cool (Symrise) erhältlich.

Der Gewichtsanteil des Kühlwirkstoffs am Gesamtgewicht der kosmetischen Zusammensetzung beträgt vorzugsweise 0,01 bis 1,0 Gew.-%, bevorzugt 0,02 bis 0,6 Gew.-% und insbesondere 0,05 bis 0,4 Gew.-%.

Die Zusammensetzung einiger weiterer bevorzugter kosmetischer Zusammensetzungen kann der folgenden Übersicht 5 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 9,0 bis 15 | 9,0 bis 14 |
| Treibmittel | 80 bis 94 | 80 bis 94 | 80 bis 92 | 80 bis 90 | 80 bis 90 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophobierte oder hydrophilierte Faser natürlichen Ursprungs | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophobierte oder hydrophilierte Cellulosefaser | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophobierte oder hydrophilierte Faser natürlichen Ursprungs mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophobierte oder hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Stärkeverbindung | 1,0 bis 10 | 1,0 bis 10 | 2,0 bis 6,0 | 2,0 bis 6,0 | 3,0 bis 5,0 |
| Kühlwirkstoff | 0,01 bis 1,0 | 0,02 bis 0,6 | 0,02 bis 0,6 | 0,05 bis 0,4 | 0,05 bis 0,4 |
| Ethanol | 5,0 bis 19 | 5,0 bis 19 | 7,0 bis 17 | 8,0 bis 15 | 8,0 bis 12 |
| Treibmittel | 78 bis 92 | 78 bis 90 | 78 bis 88 | 78 bis 88 | 78 bis 88 |
| Optionale Additive | ad 100 | ad 100 | ad 100 | ad 100 | ad 100 |

In einer weiteren alternativen Ausführungsform bzw. in Kombination mit den vorgenannten hydrophobierten oder hydrophilierten Fasern enthalten bevorzugte kosmetische Zusammensetzungen mindestens einen Duftstoff.

Der weiterer optionaler Bestandteil der kosmetischen Zusammensetzung sind Polymere einsetzbar. Für die Applizierbarkeit und kosmetische Wirkung der Zusammensetzungen hat es sich als vorteilhaft erwiesen, den Gewichtsanteil des Polymers am Gesamtgewicht der Zusammensetzung auf 0,1 bis 4,0 Gew.-%, vorzugsweise 0,1 bis 2,0 Gew.-% und insbesondere 0,1 bis 1,0 Gew.-% zu begrenzen.

Polymere werden in den kosmetischen Zusammensetzungen beispielsweise aufgrund ihrer festigenden und/oder filmbildenden Eigenschaften eingesetzt. Aufgrund ihrer kosmetischen Wirkung ist der Einsatz filmbildender Polymere besonders bevorzugt.

Als Polymere eignen sich permanent als auch temporär kationische, anionische, nichtionische oder amphotere Polymere. Die filmbildenden Polymere können synthetischen oder natürlichen Ursprungs sein.

Beispiele für gebräuchliche Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Mit besonderem Vorzug ist das Polymer ausgewählt aus der Gruppe der amphoteren Polymere. Ganz besonders bevorzugt ist der Einsatz eines Copolymers von i) N-tert-Octylacrylamid, ii) Acrylsäure, iii) tert.-Butylaminoethylmethacrylat sowie gegebenenfalls weiteren Monomeren

Bevorzugte amphotere Copolymere bestehen bevorzugt zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylat. Entsprechende Copolymere können auch unter ausschließlicher Nutzung der Monomere N-tert-Octylacrylamid, Acrylsäure und tert.-Butylaminoethylmethacrylat erhalten werden.

Bevorzugt sind weiterhin Copolymere aus den Monomeren N-tert-Octylacrylamid, Acrylsäure, tert.-Butylaminoethylmethacrylat, Methylmethacrylat und Hydroxypropylmethacrylat. Besonders bevorzugt ist es insbesondere, wenn das Copolymer zu mindestens 90 Gew.-%, vorzugsweise zu mindestens 95 Gew.-% und insbesondere zu mindestens 97 Gew.-% aus den Monomeren N-tert-Octylacrylamid, Acrylsäure, tert.-Butylaminoethylmethacrylat, Methylmethacrylat und Hydroxypropylmethacrylat besteht.

Die zuvor beschriebenen amphoteren Copolymere werden beispielsweise unter der Bezeichnung Amphomer^{®} (INCI-Bezeichnung: Octylacrylamide / Acrylates / Butylaminoethyl Methacrylate Copolymer; CAS Nummer 70801-07-9) von der Firma National Starch vertrieben.

Als optionale weitere geeignete Wirk- oder Hilfsstoffe, welche in den kosmetischen Zusammensetzungen enthalten sein können, sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Zur Abgabe und Applikation der kosmetischen Zusammensetzungen eignen sich insbesondere Aerosolabgabebehälter. Ein weiterer Anmeldungsgegenstand sind dem entsprechend kosmetische Mittel, umfassend
a) eine erfindungsgemäßen kosmetische Zusammensetzung
b) einen Aerosolabgabebehälter.

Unter der Bezeichnung Aerosolabgabebehälter sind Druckbehälter zu verstehen, in deren Inneren ein höherer Gasdruck herrscht als außerhalb des Behälters und aus dem sich ein Gasstrom über ein Ventil entnehmen lässt. Bei den Aerosolabgabebehälter, handelt es sich mit anderen Worten um Druckbehälter, mit deren Hilfe ein Produkt (z.B. eine kosmetische Zusammensetzung) durch den inneren Gasdruck des Behälters über ein Ventil abgegeben werden kann.

Die erfindungsgemäßen kosmetischen Mittel lassen sich in üblicher Art und Weise herstellen. In der Regel werden alle Bestandteile der kosmetischen Zusammensetzung mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge des Treibmittels eingefüllt.

Als druckfeste Behälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der im Druckbehälter konfektionierten Zusammensetzung. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Als Aerosolabgabebehälter kann auch ein Mehrkammerspender eingesetzt werden. Der Mehrkammerspender kann auch so ausgestaltet sein, dass eine Kammer das komprimierte Treibmittel und eine andere Kammer mit den restlichen Bestandteilen der erfindungsgemäßen Zusammensetzung befüllt ist. Ein derartiger Mehrkammerspender ist beispielsweise eine so genannte Bag-in-Can-Verpackung.

Die Sprührate der kosmetischen Mittel beträgt bevorzugt 6,5 bis 10,0 g/10 s.

Wie weiter oben ausgeführt, eignen sich die die erfindungsgemäßen Mittel insbesondere zur Reinigung, Auffrischung und Desodorierung keratinischer Fasern. Entsprechende Verwendungen der kosmetischen Zusammensetzungen sind weitere Gegenstände dieser Anmeldung.

Ein letzter Gegenstand dieser Anmeldung ist ein Verfahren zur temporären Verformung von keratinischen Fasern, insbesondere menschlichen Haaren, bei welchem eine erfindungsgemäße kosmetische Zusammensetzung auf keratinische Fasern appliziert wird. Für die Bestandteile dieser Zusammensetzung, deren Gewichtsanteile und bevorzugte Ausführungsformen gilt mutatis mutandis das zuvor Gesagte.

## Patentansprüche

1. Eine kosmetische Zusammensetzung zur kosmetischen Behandlung keratinischer Fasern, umfassend- jeweils bezogen auf ihr Gesamtgewicht -:
(a) 0, 1 bis 2,0 Gew.-% Fasern
(b) 5,0 bis 19 Gew.-% Ethanol
(c) 78 bis 94 Gew.-% Treibmittel,
wobei die Zusammensetzung 0,1 bis 2,0 Gew.-% hydrophobierte oder hydrophilierte Fasern enthält.

2. Kosmetische Zusammensetzung nach Anspruch 1, wobei sie ferner
(d) 0 bis 5 Gew.-% Farbstoff(e)
(e) 0 bis 5 Gew.-% Pigment(e)
enthält, mit der Maßgabe, dass
die Summe der Mengen der Inhaltsstoffe (d) und (e) 0,0001 bis 10 Gew.-%, bezogen auf das Gesamtgewicht der kosmetischen Zusammensetzung, beträgt.

3. Kosmetische Zusammensetzung nach Anspruch 1 oder 2, wobei der Gewichtsanteil der Fasern am Gesamtgewicht der kosmetischen Zusammensetzung 0,1 bis 1 ,0 Gew.-% und insbesondere 0,1 bis 0,5 Gew.-% beträgt.

4. Kosmetische Zusammensetzung nach einem der vorherigen Ansprüche, wobei die Fasern ausgewählt sind aus der Gruppe der aus natürlichen Fasermaterialien hergestellten Fasern.

5. Kosmetische Zusammensetzung nach einem der vorherigen Ansprüche, wobei die Fasern ausgewählt sind aus der Gruppe der pflanzlichen Fasern, insbesondere der Cellulosefasern, vorzugsweise der Baumwollfasern, Flachsfasern und Kapopkfasern.

6. Kosmetische Zusammensetzung nach einem der vorherigen Ansprüche, wobei der Gewichtsanteil des Ethanols am Gesamtgewicht der kosmetischen Zusammensetzung 7,0 bis 17 Gew.-%, vorzugsweise 9,0 bis 15 Gew.-% und insbesondere 10 bis 14 Gew.-% beträgt.

7. Kosmetische Zusammensetzung nach einem der vorherigen Ansprüche, wobei die Zusammensetzung als weiteren Bestandteil mindestens eine Stärkeverbindung umfasst.

8. Kosmetische Zusammensetzung nach einem der vorherigen Ansprüche, wobei die Zusammensetzung bezogen auf ihr Gesamtgewicht 1 ,0 bis 10 Gew.-%, vorzugsweise 2,0 bis 6,0 Gew.-% und insbesondere 3,0 bis 5,0 Gew.-% einer Stärkeverbindung umfasst.

9. Kosmetische Zusammensetzung nach einem der vorherigen Ansprüche 2 bis 7, wobei sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf ihr Gesamtgewicht, Farbstoff(e) enthält.

10. Kosmetische Zusammensetzung nach einem der vorherigen Ansprüche 2 bis 8, wobei sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf ihr Gesamtgewicht, Pigment(e) enthält.

11. Kosmetische Zusammensetzung nach einem der vorherigen Ansprüche 2 bis 9, wobei sie mindestens ein Pigment aus der Gruppe CI 12490, CI 14700, CI 14720, CI15510, CI15985, CI45380, CI47005, CI60730, CI61565, CI73360, CI74160, CI77007, CI77019, CI77288, CI77289, CI77491 , CI77492 enthält, wobei die Gesamtmenge an Pigment(en) aus dieser Gruppe 0,0001 bis 10 Gew.- %, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf das Gesamtgewicht der kosmetischen Zusammensetzung, beträgt.

12. Kosmetische Zusammensetzung nach Anspruch 2 und einem der vorherigen Ansprüche 6 bis 10, wobei sie den Inhaltsstoff (d) und/oder (e) in Form eines Stärke-Farbstoff-Compounds und/oder Stärke-Pigment-Compounds, vorzugsweise in Form von gefärbter und/oder pigmentierter Reisstärke, enthält.

13. Kosmetische Zusammensetzung nach einem der vorherigen Ansprüche 2 bis 11, wobei sie die Inhaltsstoffe (a) und (d) und/oder (a) und (e) in Form eines Faser-Farbstoff-Compounds oder Faser- Pigment-Compounds, vorzugsweise in Form von gefärbten und/oder pigmentierten Fasern, enthält.

14. Kosmetisches Mittel, umfassend
a) eine kosmetische Zusammensetzung nach einem der Ansprüche 1 bis 12
b) einen Aerosolabgabebehälter.

15. Verwendung einer kosmetischen Zusammensetzung nach einem der Ansprüche 1 bis 12 zur Reinigung keratinischer Fasern.

## Claims

1. A cosmetic composition for the cosmetic treatment of keratin fibers, comprising, in each case based on its total weight:
(a) 0.1 to 2.0 wt.% fibers
(b) 5.0 to 19 wt.% ethanol
(c) 78 to 94 wt.% propellant,
wherein the composition contains 0.1 to 2.0 wt.% hydrophobic or hydrophilic fibers.

2. Cosmetic composition according to claim 1, wherein it further contains
(d) 0 to 5 wt.% dye(s)
(e) 0 to 5 wt.% pigment(s)
with the proviso that
the sum of the amounts of ingredients (d) and (e) is 0.0001 to 10 wt.%, based on the total weight of the cosmetic composition.

3. Cosmetic composition according to claim 1 or 2, wherein the proportion by weight of the fibers with respect to the total weight of the cosmetic composition is 0.1 to 1.0 wt.% and in particular 0.1 to 0.5 wt.%.

4. Cosmetic composition according to one of the preceding claims, wherein the fibers are selected from the group of fibers made from natural fiber materials.

5. Cosmetic composition according to one of the preceding claims, wherein the fibers are selected from the group of vegetable fibers, in particular cellulose fibers, preferably cotton fibers, flax fibers and kapok fibers.

6. Cosmetic composition according to one of the preceding claims, wherein the proportion by weight of the ethanol with respect to the total weight of the cosmetic composition is 7.0 to 17 wt.%, preferably 9.0 to 15 wt.%, and in particular 10 to 14 wt.%.

7. Cosmetic composition according to one of the preceding claims, wherein the composition comprises at least one starch compound as a further constituent.

8. Cosmetic composition according to one of the preceding claims, wherein the composition comprises, based on its total weight, 1.0 to 10 wt.%, preferably 2.0 to 6.0 wt.%, and in particular 3.0 to 5.0 wt.%, of a starch compound.

9. Cosmetic composition according to one of the preceding claims 2 to 7, wherein it contains 0.0001 to 10 wt.%, preferably 0.01 to 9.5 wt.%, more preferably 0.05 to 9 wt.%, even more preferably 0.1 to 8.5 wt.% and in particular 0.25 to 8 wt.%, in each case based on its total weight, dye(s).

10. Cosmetic composition according to one of the preceding claims 2 to 8, wherein it contains 0.0001 to 10 wt.%, preferably 0.01 to 9.5 wt.%, more preferably 0.05 to 9 wt.%, even more preferably 0.1 to 8.5 wt.% and in particular 0.25 to 8 wt.%, in each case based on its total weight, pigment(s).

11. Cosmetic composition according to one of the preceding claims 2 to 9, wherein it contains at least one pigment from the group CI 12490, CI 14700, CI 14720, C115510, CI15985, C145380, CI47005, CI60730, C161565, C173360, C174160, CI77007, C177019, C177288, C177289, C177491, C177492, wherein the total amount of pigment(s) from this group is 0.0001 to 10 wt.%, preferably 0.01 to 9.5 wt.%, more preferably 0.05 to 9 wt.%, even more preferably 0.1 to 8.5 wt.% and in particular 0.25 to 8 wt.%, in each case based on the total weight of the cosmetic composition.

12. Cosmetic composition according to claim 2 and one of the preceding claims 6 to 10, wherein it contains ingredient (d) and/or (e) in the form of a starch-dye compound and/or starch-pigment compound, preferably in the form of colored and/or pigmented rice starch.

13. Cosmetic composition according to one of the preceding claims 2 to 11, wherein it contains ingredients (a) and (d) and/or (a) and (e) in the form of a fiber-dye compound or fiber-pigment compound, preferably in the form of colored and/or pigmented fibers.

14. Cosmetic agent, comprising
a) a cosmetic composition according to one of claims 1 to 12
b) an aerosol dispensing container.

15. Use of a cosmetic composition according to one of claims 1 to 12 for cleaning keratin fibers.

## Revendications

1. Composition cosmétique pour le traitement cosmétique de fibres kératiniques, comprenant, respectivement par rapport à son poids total :
(a) 0,1 à 2,0 % en poids de fibres
(b) 5,0 à 19 % en poids d'éthanol
(c) 78 à 94 % en poids d'agent propulseur,
dans laquelle la composition contient 0,1 à 2,0 % en poids de fibres hydrophobes ou hydrophilisées.

2. Composition cosmétique selon la revendication 1, dans laquelle elle contient en outre
(d) 0 à 5 % en poids de colorant(s)
(e) 0 à 5 % en poids de pigment(s)
à condition que
la somme des quantités des ingrédients (d) et (e) aille de 0,0001 à 10 % en poids, par rapport au poids total de la composition cosmétique.

3. Composition cosmétique selon la revendication 1 ou 2, dans laquelle la proportion en poids des fibres par rapport au poids total de la composition cosmétique va de 0,1 à 1,0 % en poids et en particulier de 0,1 à 0,5 % en poids.

4. Composition cosmétique selon l'une des revendications précédentes, dans laquelle les fibres sont choisies dans le groupe des fibres constituées de matériaux fibreux naturels.

5. Composition cosmétique selon l'une des revendications précédentes, dans laquelle les fibres sont choisies dans le groupe des fibres végétales, en particulier des fibres de cellulose, de préférence des fibres de coton, des fibres de lin et des fibres de kapok.

6. Composition cosmétique selon l'une des revendications précédentes, dans laquelle la proportion en poids d'éthanol par rapport au poids total de la composition cosmétique va de 7,0 à 17 % en poids, de préférence de 9,0 à 15 % en poids et en particulier de 10 à 14 % en poids.

7. Composition cosmétique selon l'une des revendications précédentes, dans laquelle la composition comprend, en tant que constituant supplémentaire, au moins un composé d'amidon.

8. Composition cosmétique selon l'une des revendications précédentes, dans laquelle la composition comprend, par rapport à son poids total, 1,0 à 10 % en poids, de préférence 2,0 à 6,0 % en poids et en particulier 3,0 à 5,0 % en poids d'un composé d'amidon.

9. Composition cosmétique selon l'une des revendications précédentes 2 à 7, dans laquelle elle contient 0,0001 à 10 % en poids, de préférence 0,01 à 9,5 % en poids, plus préférentiellement 0,05 à 9 % en poids, encore plus préférentiellement 0,1 à 8,5 % en poids et en particulier 0,25 à 8 % en poids de colorant(s), respectivement par rapport à son poids total.

10. Composition cosmétique selon l'une des revendications précédentes 2 à 8, dans laquelle elle contient 0,0001 à 10 % en poids, de préférence 0,01 à 9,5 % en poids, plus préférentiellement 0,05 à 9 % en poids, encore plus préférentiellement 0,1 à 8,5 % en poids et en particulier 0,25 à 8 % % en poids de pigment(s), respectivement par rapport à son poids total.

11. Composition cosmétique selon l'une des revendications précédentes 2 à 9, dans laquelle elle contient au moins un pigment du groupe CI 12490, CI 14700, CI 14720, CI 15510, CI 15985, CI 45380, CI 47005, CI 60730, CI 61565, CI 73360, CI 74160, CI 77007, CI 77019, CI 77288, CI 77289, CI 77491, CI 77492, dans laquelle la quantité totale de pigment(s) provenant dudit groupe va de 0,0001 à 10 % en poids, de préférence de 0,01 à 9,5 % en poids, plus préférentiellement de 0,05 à 9 % en poids, encore plus préférentiellement de 0,1 à 8,5 % en poids et en particulier de 0,25 à 8 % % en poids, respectivement par rapport au poids total de la composition cosmétique.

12. Composition cosmétique selon la revendication 2 et l'une des revendications précédentes 6 à 10, dans laquelle elle contient l'ingrédient (d) et/ou (e) sous forme de composé amidon/colorant et/ou de composé amidon/pigment, de préférence sous forme d'amidon de riz coloré et/ou pigmenté.

13. Composition cosmétique selon l'une des revendications précédentes 2 à 11, dans laquelle elle contient les ingrédients (a) et (d) et/ou (a) et (e) sous forme de composé fibre/colorant ou de composé fibre/pigment, de préférence sous forme de fibres colorées et/ou pigmentées.

14. Agent cosmétique, comprenant
a) une composition cosmétique selon l'une des revendications 1 à 12
b) un récipient de distribution aérosol.

15. Utilisation d'une composition cosmétique selon l'une des revendications 1 à 12 pour le nettoyage de fibres kératiniques.
